# EUROPÄISCHE PATENTSCHRIFT

(11) **EP 1 154 752 B1**
(45) Veröffentlichungstag und Bekanntmachung des Hinweises auf die Patenterteilung: **26.10.2005**
(21) Anmeldenummer: 00910673.3
(22) Anmeldetag: 12.02.2000
(51) Int. Cl.: A61K 7/06

(54) **ZUBEREITUNGEN ZUR BEHANDLUNG KERATINISCHER FASERN**
PREPARATIONS FOR TREATING KERATIN FIBRES
PREPARATIONS DESTINEES AU TRAITEMENT DE FIBRES KERATINIQUES

(30) Priorität: 23.02.1999 DE 19907714
(43) Veröffentlichungstag der Anmeldung: 21.11.2001
(73) Patentinhaber: Hans Schwarzkopf & Henkel GmbH & Co. KG, 22763 Hamburg (DE)
(72) Erfinder: SCHRÖDER, Thomas, D-22523 Hamburg (DE); BERNECKER, Ullrich, D-52393 Hürtgenwald (DE); POPPE, Elisabeth, D-22299 Hamburg (DE); KRÜGER, Marcus, D-22763 Hamburg (DE)
(74) Vertreter: Mathes, Nikolaus
(86) Internationale Anmeldenummer: PCT/EP2000/001159
(87) Internationale Veröffentlichungsnummer: WO 2000/050000

(56) Entgegenhaltungen:
- EP-A- 0 277 876
- EP-A- 0 723 770
- EP-A- 0 875 236
- WO-A-96/32921
- DE-A- 3 620 849

## Beschreibung

Die Erfindung betrifft Zubereitungen zur Behandlung keratinischer Fasern, insbesondere menschlicher Haare, mit einer speziellen Wirkstoffkombination, die Verwendung dieser Zubereitungen zur Behandlung keratinischer Fasern sowie ein Verfahren zur Behandlung keratinischer Fasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Wellmitteln, Färbemitteln, Tönungsmitteln und Stylingpräparaten.

Dabei kommt der Pflege der Haare eine ständig wachsende Bedeutung zu. Dies betrifft nicht allein die seit jeher vornehmlich der Pflege der Haare dienenden Mittel wie Haarkuren und Haarspülungen. Auch für die Vermarktung von Produkten, die in erster Linie beispielsweise der Reinigung, Färbung oder Formgebung der Haare dienen, ist es zunehmend wichtig, pflegende Wirkungen ausloben zu können.

Schließlich ist es bei der zunehmenden Komplexität der Haarbehandlungsmittel wichtig, wenn die neu gefundenen Wirkstoffe oder Wirkstoffkombinationen sich durch hohe Kompatibilität mit möglichst vielen gängigen Bestandteilen von Haarbehandlungsmitteln auszeichnen oder sogar bisherige Kompatibilitätsprobleme umgehbar machen.

Unter Pflege von Haaren ist im weitesten Sinne sowohl die Reparatur, Minderung oder Prävention von Haarschädigungen, wie beispielsweise Brüchen, Oberflächendefekten und Spliß, als auch die Verringerung oder Vermeidung unerwünschter Eigenschaften des Haares, wie beispielsweise schlechte Naßkämmbarkeit, Verknotung der Haare, geringer Glanz und erhöhte elektrostatische Aufladung, zu verstehen.

Zur Pflege der Haare werden beispielsweise quaternäre Ammoniumverbindungen, kationische und nichtionogene Polymere, Silikone sowie Proteinhydrolysate und Aminosäuren eingesetzt. Bezüglich Einzelheiten wird ausdrücklich auf die bekannten Handbücher wie Umbach, Kosmetik, 2. Auflage, Georg Thieme Verlag Stuttgart New York, 1995, und Kh. Schrader, Grundlagen und Rezepturen der Kosmetika, 2. Auflage, Hüthig Buch Verlag, Heidelberg, 1989, verwiesen.

Dennoch sind die bisherigen Zubereitungen nicht bezüglich aller Parameter optimal zu nennen; häufig müssen auch aus Kompatibilitätsgründen Kompromisse eingegangen werden. Es besteht daher weiterhin ein Bedarf an neuen Pflegewirkstoffen und Kombinationen bekannter Wirkstoffe, die sich durch stark verbesserte oder zusätzliche Eigenschaften auszeichnen.

Es wurde nun überraschenderweise gefunden, daß durch eine spezielle Wirkstoffkombination nicht nur die Pflegeleistung unerwartet stark gesteigert wird, sondern sich auch schwerwiegende Kompatibilitätsprobleme umgehen lassen, die in vielen Fällen bei der Verwendung der üblicherweise bevorzugt eingesetzten anionischen Verdickungsmittel auftreten würden. Weiterhin hat sich gezeigt, daß bei Verwendung dieser Wirkstoffkombination in Haarfärbemitteln bzw. in direkt nach der Färbung angewendeten Nachbehandlungsmitteln überraschenderweise eine bessere Waschechtheit der Färbung erhalten wird.

Gegenstand der Erfindung sind daher Zubereitungen zur Behandlung keratinischer Fasern, insbesondere menschlicher Haare, dadurch gekennzeichnet, daß sie eine Wirkstoffkombination, bestehend aus einem verdickend wirkenden synthetischen Polymer (A), ausgewählt aus den Homopolymeren der allgemeinen Formel (I), 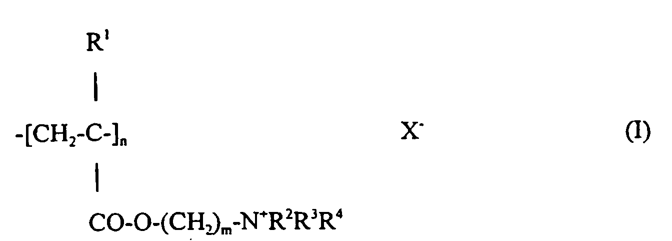 in der R¹ = -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymeren, bestehend im wesentlichen aus den in Formel (I) aufgeführten Monomereinheiten sowie nichtionogenen Monomereinheiten,
und
mindestens einem kationischen Polymer (B),
enthalten mit der Maßgabe, daß die Zubereitungen zwingend ein Tensid enthalten, wenn das Polymer (A) ein Copolymer ist.

Unter keratinischen Fasern werden erfindungsgemäß Pelze, Wolle, Federn und insbesondere menschliche Haare verstanden.

Die erste zwingende Komponente der erfindungsgemäßen Wirkstoffkombination ist ein verdickend wirkendes synthetisches Polymer (A).

Die Eigenschaft "verdickend wirkend" des Polymers (A) ist im Rahmen der vorliegenden Erfindung wie folgt definiert: Die Zugabe von 0,5 Gewichtsteilen des Polymers zu 100 Gewichtsteilen entsalztem Wasser bewirkt eine Erhöhung der Viskosität auf mindestens den 50fachen Wert, bezogen auf die Viskosität des entsalzten Wassers. Die Bestimmung der Viskosität wird bei 22 °C mit einem Viskosimeter der Marke Brookfield RVT durchgeführt (Speed: 50 Umdrehungen pro Minute; Spindel 1 bei Viskositäten bis zu 200 mPas, Spindel 3 bei Viskositäten von 200-2000 mPas oder Spindel 5 bei Viskositäten von über 2000 mPas; Ablesen des Meßwertes jeweils eine Minute nach Beginn der Messung). Polymere (A), die eine Erhöhung der Viskosität auf mindestens den 200fachen Wert bewirken, sind bevorzugt.

Im Rahmen der Gruppe der Polymere (A), die Homopolymere gemäß Formel (I) oder Copolymere sind, die aus den in Formel (I) aufgeführten Monomereinheiten sowie nichtionogenen Monomereinheiten bestehen, sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2,

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Gemäß einer ersten, besonders bevorzugten, Ausführungsform werden als Polymere (A) Homopolymere gemäß Formel (I) eingesetzt. Im Rahmen dieser Ausführungsform bevorzugt ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethem von Zuckern oder Zuckerderivaten wie Erythritol, Pentaerythritol, Arabitol, Monnitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50% Polymeranteil, weitere Komponenten: Mineralöl (INCI-Bezeichnung: Mineral Oil) und Tridecylpolyoxypropylen-polyoxyethylen-ether (INCI-Bezeichnung: PPG-1-Trideceth-6)) und Salcare® SC 96 (ca. 50% Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylenpolyoxyethylen-ether (INCI-Bezeichnung: PPG-1-Trideceth-6) im Handel erhältlich.

Gemäß einer zweiten, bevorzugten, Ausführungsform werden als Polymere (A) Copolymere eingesetzt, deren nichtionogene Monomereinheiten ausgewählt sind aus der Gruppe, die von Acrylamid, Methacrylamid, Acrylsäure-C₁₋₄-Alkylestern und Methacrylsäure-C₁₋₄-Alkylestern gebildet wird. Unter diesen nichtionogenen Monomeren ist das Acrylamid besonders bevorzugt. Auch diese Copolymere können, wie im Falle der Homopolymeren oben beschrieben, vemetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomeren in einem Gewichtsverhältnis von 20:80 vorliegen, sind im Handel als ca. 50 %ige nichtwäßrige Polymerdispersion unter der Bezeichnung Salcare® SC 92 erhältlich.

Weitere Informationen über die Produkte Salcare® SC 95, Salcare® SC 96 und Salcare® SC 92 sind den Patentschriften EP-B1-0 686 024 und EP-B1-0 761 206, ferner den Druckschriften "Richtrezepturen Salcare SC 95", "Richtrezepturen Salcare SC 96" sowie "Salcare SC 95 (TPD 6237)" und Salcare SC 96 (TPD 6252)" der Allied Colloids GmbH zu entnehmen. Diese Druckschriften enthalten jedoch keinerlei Hinweise auf die erfindungsgemäßen synergistischen Wirkstoffkombinationen.

Die zweite zwingende Komponente der erfindungsgemäßen Wirkstoffkombination ist ein kationisches Polymer (B).

Als kationisches Polymer (B) kommen alle für die Haarbehandlung bekannten kationischen Polymeren in Betracht.

Eine erste Gruppe von kationischen Polymeren sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Bevorzugt ist aber, die kationischen Polymere (B) aus den sogenannten "permanent kationischen" Polymeren auszuwählen. Diese Polymere enthalten üblicherweise quartäre Ammoniumgruppen, die unabhängig vom pH-Wert der Zubereitung kationisch vorliegen.

Bevorzugte kationische Polymere sind beispielsweise
- kationische Cellulose-Derivate, wie sie unter den Bezeichnungen Celquat® und Polymer JR® im Handel erhältlich sind. Die Handelsprodukte Celquat® H 100, Celquat® L 200 und Polymer JR®400 sind bevorzugte quaternierte Cellulose-Derivate.
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere.
- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminoalkylmethacrylat-Copolymere. Solche Verbindungen sind unter den Bezeichnungen Gafquat®734 und Gafquat®755 im Handel erhältlich.
- Vinylpyrrolidon-Vinylimidazoliummethochlorid-Copolymere, wie sie unter der Bezeichnung Luviquat® angeboten werden.
- quaternierter Polyvinylalkohol
- die unter den INCI-Bezeichnungen Polyquaternium-2, Polyquaternium-17, Polyquaternium-18 und Polyquaternium-27 bekannten Polymeren mit quartären Stickstoffatomen in der Polymerhauptkette, die unter dem Warenzeichen Mirapol® im Handel sind.

Erfindungsgemäß besonders bevorzugte Polymere (B) sind die kationischen Cellulose-Derivate, insbesondere das Handelsprodukt Polymer JR® 400, sowie das Handelsprodukt Gafquat®755.

Kationische Polymere (B) im Sinne der Erfindung sind auch kationisch derivatisierte Proteinhydrolysate, wie sie beispielsweise unter den Warenzeichen Lamequat® und Croquat® im Handel erhältlich sind.

Im Rahmen einer ersten bevorzugten Ausführungsform der Erfindung weist das gewählte Polymer (B) keine verdickenden Eigenschaften gemäß der oben stehenden Definition auf.

Im Rahmen der erfindungsgemäßen Lehre ist es aber auch möglich, zusätzlich zu dem Polymer (A) ein von diesem strukturell verschiedenes kationisches Polymer (B) einzusetzen, das ebenfalls verdickend wirkt.

Ebenfalls von der erfinderischen Lehre umfaßt ist der Einsatz von mehr als einem, insbesondere von zwei, Polymeren (A) und/oder der Einsatz von mehr als einem, insbesondere von zwei, kationischen Polymeren (B).

Die Polymere (A) sind in den erfindungsgemäßen Zubereitungen in Mengen von 0,1 - 10 Gew.-%, insbesondere von 0,5 - 5 Gew.-%, bezogen auf die gesamte Zubereitung, enthalten.

Die Polymere (B) sind in den erfindungsgemäßen Zubereitungen in Mengen von 0,01 - 5 Gew.-%, insbesondere von 0,02 - 3 Gew.-%, bezogen auf die gesamte Zubereitung, enthalten.

Gemäß einer bevorzugten Ausführungsform enthalten die erfindungsgemäßen Zubereitungen weiterhin mindestens ein Silikon.

Erfindungsgemäß geeignete Silikone sind insbesondere Dialkyl- und Alkylarylsiloxane, wie beispielsweise Dimethylpolysiloxan und Methylphenyl-polysiloxan, sowie deren alkoxylierte und quaternierte Analoga.
Beispiele für solche Silikone sind:
- Oligomere Polydimethylcyclosiloxane (INCI-Bezeichnung: Cyclomethicone), insbesondere die tetramere und die pentamere Verbindung, die als Handelsprodukte DC 344 bzw. DC 345 von Dow Corning vertrieben werden,
- Hexamethyl-Disiloxan (INCI-Bezeichnung: Hexamethyldisiloxane), z. B. das unter der Bezeichnung Abil® K 520 vertriebenen Produkt,
- Polymere Polydimethylsiloxane (INCI-Bezeichnung: Dimethicone), z. B. die unter der Bezeichnung DC 200 von Dow Coming vertriebenen Produkte,
- Polyphenylmethylsiloxane (INCI-Bezeichnung: Phenyl Trimethicone), z. B. das Handelsprodukt DC 556 Fluid von Dow Corning,
- Silicon-Glykol-Copolymere (INCI-Bezeichnung: Dimethicone Copolyol), z. B. die Handelsprodukte DC 190 und DC 193 von Dow Corning,
- Dimethylsiloxane mit Hydroxy-Endgruppen (INCI-Bezeichnung: Dimethiconol), z. B. die Handelsprodukte DC 1401 und Q2-1403 von Dow Corning,
- aminofunktionelle Polydimethylsiloxane und hydroxylaminomodifizierte Silicone (INCI-Bezeichnung: u. a. Amodimethicone und Quaternium-80), wie die Handelsprodukte Dow Corning® 929 Emulsion, Dow Corning® 939, SM-2059 (Hersteller: General Electric), SLM-55067 (Hersteller: Wacker) sowie Abil®-Quat 3270 und 3272 (Hersteller: Th. Goldschmidt).

Erfindungsgemäß können sowohl wasserlösliche als auch wasserunlösliche Silikone eingesetzt werden. Als wasserunlöslich werden solche Silikone bezeichnet, die bei 20 °C zu weniger als 0,1 Gew.-% in Wasser löslich sind. Es kann erfindungsgemäß bevorzugt sein, mindestens ein wasserunlösliches Silikon einzusetzen.

Weiterhin können sowohl flüchtige als auch nichtflüchtige Silikone eingesetzt werden. Flüchtig im Sinne der Erfindung sind solche Silikone, die ein Flüchtigkeit aufweisen, die gleich oder größer als die Flüchtigkeit des cyclischen, pentameren Dimethylsiloxans ist.

Gemäß einer besonders bevorzugten Ausführungsform enthalten die erfindungsgemäßen Zubereitungen eine Kombination aus einem flüchtigen und einem nichtflüchtigen Silikon. Solche Kombinationen sind auch als Handelsprodukte (z. B. Dow Corning®1401, Dow Corning®1403 und Dow Corning®1501, jeweils Mischungen aus einem Cyclomethicone und einem Dimethiconol) erhältlich.

Die erfindungsgemäßen Zubereitungen enthalten die Silikone bevorzugt in Mengen von 0,05 - 10 Gew.-%, insbesondere 0,1 - 5 Gew.-%, bezogen auf die gesamte Zubereitung. In Einzelfällen, z. B. bei Formulierung als Haarspitzenfluid, kann die Silikon-Menge aber auch bis zu 70 Gew.-% betragen.

Bei Verwendung einer Kombination von nichtflüchtigen und flüchtigen Silikonen liegen diese bevorzugt in einem Massenverhältnis von 1:2 bis 1:10, insbesondere von 1:4 bis 1:8, vor.

Gemäß einer weiteren bevorzugten Ausführungsform enthalten die erfindungsgemäßen Zubereitungen weiterhin ein Proteinhydrolysat. Proteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Henkel), Promois® (Interorgana), Collapuron® (Henkel), Nutrilan® (Grünau), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin® (Henkel), DiaMin® (Diamalt), Lexein® (Inolex) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls, wenngleich weniger bevorzugt, ist der Einsatz der Proteinhydrolysate in Form ihrer Fettsäure-Kondensationsprodukte. Solche Handelsprodukte werden beispielsweise unter den Bezeichnungen Lamepon® (Henkel), Lexein® (Inolex), Crolastin® (Croda) und Crotein® (Croda) vertrieben.

Die Proteinhydrolysate sind in den erfindungsgemäßen Zubereitungen bevorzugt in Mengen von etwa 0,01 - 10 Gew.-%, bezogen auf die gesamte Zubereitung, enthalten

Gemäß einer weiteren bevorzugten Ausführungsform enthalten die erfindungsgemäßen Zubereitungen weiterhin mindestens ein Tensid. Erfindungsgemäß bevorzugt sind dabei kationische Tenside, insbesondere vom Typ der quartären Ammoniumverbindungen, der Esterquats und der Amidoamine.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quatemium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Je nach Verwendungszweck der Zubereitung kann diese auch noch Tenside anderer Tensidklassen enthalten.

Geeignete nichtionogene Tenside sind beispielsweise Alkylphenolpolyglycolether, Fettsäurepolyglycolester, Fettsäureamidpolygylcolether, Fettaminpolyglycolether, alkoxylierte Triglyceride, wie insbesondere ethoxyliertes Rizinusöl, Alk(en)yloligoglucoside, Fettsäure-N-alkylglucamide, Polyolfettsäureester, Zuckerester, Sorbitanester und Polysorbate. Sofern die nichtionischen Tenside Polyglycoletherketten enthalten, können diese eine konventionelle oder eingeengte Homologenverteilung aufweisen.

Geeignete anionische Tenside sind insbesondere Alkylsulfate, Alkylpolyglykolethersulfate und Ethercarbonsäuren mit 10 bis 18 C-Atomen in der Alkylgruppe und bis zu 12 Glykolethergruppen im Molekül, Seifen sowie Sulfobernsteinsäuremono- und - dialkylester mit 8 bis 18 C-Atomen in der Alkylgruppe und Sulfobernsteinsäuremonoalkylpolyoxyethyl-ester mit 8 bis 18 C-Atomen in der Alkylgruppe und 1 bis 6 Oxyethylgruppen.

Geeignete zwitterionische Tenside sind insbesondere die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.

Geeignete ampholytische Tenside sind beispielsweise N-Alkylglycine, N-Alkylpropionsäuren, N-Alkylamino buttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkyl-aminoessigsäuren mit jeweils etwa 8 bis 18 C-Atomen in der Alkylgruppe.

Die Tenside sind in den erfindungsgemäßen Zubereitungen bevorzugt in Mengen von etwa 0,01 - 20 Gew.-%, bezogen auf die gesamte Zubereitung, enthalten.

Bezüglich der Konfektionierung der erfindungsgemäßen Zubereitungen bestehen keine prinzipiellen Einschränkungen. Üblicherweise haben die Zubereitungen eine wäßrige oder wäßrig-alkoholische Basis. Als Alkohole kommen dabei insbesondere niedere Alkohole wie Ethanol und Isopropanol in Betracht. Wäßrig-alkoholische Grundlagen können dabei Wasser und Alkohol, bevorzugt in einem Verhältnis von 1:5 bis 5:1, enthalten. Eine bevorzugte wäßrige-alkoholische Basis weist einen Alkoholgehalt von bis zu 15 Gew.-%, bezogen auf die Wassermenge, auf.

Es ist aber erfindungsgemäß auch möglich, die Zubereitungen praktisch wasserfrei, d. h. auf einer alkoholischen, z. B. Propylenglykol-, Basis zu formulieren. Diese Zubereitungen enthalten dann entweder überhaupt kein Wasser oder Wasser nur in solchen Mengen, wie sie durch andere Bestandteile aufgrund deren Konfektionierungsform eingebracht werden.

Die erfindungsgemäßen Zubereitungen können sowohl auf dem Haar verbleiben als auch nach einer Einwirkzeit, die mehrere Sekunden bis zu etwa einer Stunde beträgt, wieder ausgespült werden. Auf dem Haar verbleibende erfindungsgemäße Zubereitungen haben sich als besonders wirksam erwiesen und können daher bevorzugte Ausführungsformen der erfindungsgemäßen Lehre darstellen.

Gemäß einer ersten Ausführungsform werden die erfindungsgemäßen Zubereitungen als Haarkur, Haar-Conditioner, Haarspitzenfluid oder Haarwasser formuliert. Die erfindungsgemäßen Zubereitungen gemäß dieser Ausführungsform können nach Ablauf dieser Einwirkzeit mit Wasser oder einem zumindest überwiegend wasserhaltigen Mittel ausgespült werden; bevorzugt werden sie jedoch auf dem Haar belassen. Diese Haarkuren und Haar-Conditioner können in einer bevorzugten Variante als Schaumaerosole formuliert werden.

Dazu können die Mittel Treibgase enthalten. Bevorzugt ist in dieser Variante jedoch die Formulierung als Pumpspray mit Luft als Treibmittel.

Gemäß weiteren Ausführungsformen kann es sich bei den erfindungsgemäßen Mitteln beispielsweise um reinigende Mittel wie Shampoos, pflegende Mittel wie Spülungen, festigende Mittel wie Haarfestiger, Schaumfestiger, Haarsprays, Styling Gels und Fönwellen, dauerhaften Verformungsmitteln wie Dauerwell- und -fixiermittel sowie insbesondere im Rahmen eines Dauerwellverfahrens eingesetzte Vorbehandlungsmittel oder Nachspülungen, farbverändernde Mittel wie Blondiermittel, Oxidationsfärbemittel, insbesondere im Rahmen eines oxidativen Färbeverfahrens verwendete Oxidationsmittelzubereitungen oder Nachspülungen, Tönungsmittel auf Basis direktziehender Farbstoffe, Tönungsfestiger und Haar-Mascara handeln. Entsprechend können die Zubereitungen als Lösungen, Emulsionen, Gele, Cremes, Aerosole oder Lotionen formuliert werden. Sofern diese Mittel Komponenten enthalten, die miteinander in einer Zubereitung nicht über längere Zeit lagerstabil sind, so ist es auch möglich, die erfindungsgemäßen Zubereitungen in Form von zwei oder mehr Teil-Zubereitungen lagerstabil zu formulieren. Die zur Anwendung bestimmten Zubereitungen können dann unmittelbar vor der Anwendung durch Mischung der Teil-Zubereitungen hergestellt werden.

Der pH-Wert der erfindungsgemäßen Zubereitungen kann prinzipiell bei Werten von 2 bis 11 liegen. Er liegt bevorzugt zwischen 2 und 7, wobei Werte von 3 bis 5 besonders bevorzugt sind. Zur Einstellung dieses pH-Wertes kann praktisch jede für kosmetische Zwecke einsetzbare Säure oder Base verwendet werden. Üblicherweise werden als Säuren Genuß-säuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt. Geeignete Basen sind beispielsweise Ammoniak, Alkalihydroxide, Triethanolamin und N,N,N',N'-Tetrakis-(2-hydroxypropyl)-ethylendiamin.

Neben den erfindungsgemäß zwingend erforderlichen Bestandteilen können die erfindungsgemäßen Zubereitungen prinzipiell alle weiteren, dem Fachmann für solche kosmetischen Mittel bekannte Komponenten enthalten.

Weitere übliche Bestandteile der erfindungsgemäßen Zubereitungen können somit sein:
- Anionische, zwitterionische, amphotere und nichtionische Polymere wie beispielsweise Vinylacetat/Crotonsäure-Copolymere, Vinylpyrrolidon/Vinylacrylat-Copolymere, Vinylacetat/Butylmaleat/Isobornyl-acry-lat-Copolymere, Methylvinylether/Maleinsäureanhydrid-Copolymere und deren Ester, unvernetzte und mit Polyolen vernetzte Polyacrylsäuren, Acrylamidopropyl-trimethylammoniumchlorid/Acrylat-Copolymere, Octylacrylamid/Methyl-methacrylat/tert.Butylaminoethylmethacrylat/2-Hydroxypropylmethacrylat-Copolymere, Poly-vinylpyrrolidon, Vinylpyrrolidon/Vinylacetat-Copolymere, Vinylpyrrolidon/Di-methylaminoethylmethacrylat/Vinylcaprolactam-Terpolymere sowie gegebenfalls de-rivatisierte Celluloseether.
- symmetrische und unsymmetrische, lineare und verzweigte Dialkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether und Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, isoPentyl-n-octylether und 2-Methyl-pentyl-n-octylether.
- quaternierte Amine wie Methyl-1-alkylamidoethyl-2-alkylimidazolinium-methosulfat
- Entschäumer wie Silikone,
- Verdickungsmittel wie Agar-Agar, Guar-Gum, Alginate, Xanthan-Gum, Gelatine, Pektine, Hydroxyethylcellulose sowie Polyacrylamide und deren Copolymere,
- Strukturanten wie Maleinsäure,
- Parfümöle, Dimethylisosorbid und Cyclodextrine,
- Lösungsvermittler, wie Ethanol, Isopropanol, Ethylenglykol, Propylenglykol, Glycerin
   und Diethylenglykol,
- Farbstoffe zum Anfärben des Mittels,
- Antischuppenwirkstoffe wie Piroctone Olamine, Zink Omadine und Climbazol,
- weitere Substanzen zur Einstellung des pH-Wertes
- Wirkstoffe wie Allantoin, Panthenol, Nicotinsäureamid, Pyrrolidoncarbonsäuren, Vitamine und Bisabolol,
- Fettalkohole, insbesondere lineare und/oder gesättigte Fettalkohole mit 8 bis 22 C-Atomen, und Monoester der Fettsäuren mit Alkoholen mit 6 bis 24 C-Atomen,
- Pflanzenextrakte, insbesondere aus Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel,
- faserstrukturverbessernde Wirkstoffe, insbesondere Mono-, Di- und Oligosaccharide wie beispielsweise Glucose, Galactose, Fructose, Fruchtzucker und Lactose,
- konditionierende Wirkstoffe wie Paraffinöle, Polydecene, Squalane, pflanzliche Öle, z. B. Sonnenblumenöl, Orangenöl, Mandelöl, Weizenkeimöl und Pfirsichkernöl sowie Phospholipide, beispielsweise Sojalecithin, Ei-Lecithin und Kephaline,
- Lichtschutzmittel,
- Konsistenzgeber wie Zuckerester, Polyolester oder Polyolalkylether,
- Fette und Wachse, wie Walrat, Bienenwachs, Montanwachs und Paraffine,
- Fettsäurealkanolamide,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Komplexbildner wie EDTA, NTA, β-Alanindiessigsäure und Phosphonsäuren,
- direktziehende Farbstoffe
- sogenannte Kuppler- und Entwicklerkomponenten als Oxidationsfarbstoffvorprodukte,
- Reduktionsmittel wie z. B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat,
- Treibmittel wie Propan-Butan-Gemische, N₂O, Dimethylether, CO₂, N₂ und Luft sowie
- Antioxidantien.

Ein weiterer Gegenstand der Erfindung ist Verwendung einer Zubereitung nach einem der Ansprüche 1 bis 10 zur Behandlung keratinischer Fasern, insbesondere menschlicher Haare.

Ein dritter Gegenstand der Erfindung ist schließlich ein Verfahren zur Behandlung keratinischer Fasern, insbesondere menschlicher Haare, bei dem man eine Zubereitung gemäß einem der Ansprüche 1 bis 10 auf die Faser aufbringt und auf dieser beläßt.

### Formulierungsbeispiele

Alle Mengenangaben sind, soweit nicht anders vermerkt, Gewichtsteile.

### 1. Haarpflegeformulierung

| | |
|---|---|
| Salcare®SC 96¹ | 5,0 |
| Polymer JR®400² | 0,5 |
| Cetyltrimethylammoniumchlorid | 0,5 |
| Euxyl®K 400³ | 0,2 |
| Gluadin® W 20⁴ | 2,0 |
| Wasser, entsalzt | ad 100 |

| | |
|---|---|
| ¹ N,N,N-Trimethyl-2[(methyl-1-oxo-2-propenyl)oxy]-Ethanaminiumchlorid-Homo-polymer (50 % Aktivsubstanz; INCI-Bezeichnung: Polyquaternium-37 (and) Propylenglycol Dicaprilate/Dicaprate (and) PPG-1 Trideceth-6) (ALLIED COLLOIDS) | |
| ² quaternierte Hydroxyethylcellulose (INCI-Bezeichnung: Polyquaternium-10) (AMERCHOL) | |
| ³ 1,2-Dibrom-2,4-dicyanobutan (20 % Aktivsubstanz in Phenoxyethanol; INCI-Bezeichnung: Methyldibromo Glutaronitrile (and) Phenoxyethanol) (SCHÜLKE & MAYR) | |
| ⁴ Weizenproteinhydrolysat (20 % Aktivsubstanz in Wasser; INCI-Bezeichnung: Aqua (and) Hydrolized Wheat Protein (and) Sodium Benzoate (and) Phenoxyethanol (and) Methylparaben (and) Propylparaben) (HENKEL) | |

Die formulierung wird nach der Haarwäsche auf das Haar aufgebracht und verbleibt auf dem Haar.

### 2. Haarpflegeformulierung

| | |
|---|---|
| Salcare®SC 96 | 5,0 |
| Polymer JR®400 | 0,5 |
| Dow Corning® 939⁵ | 0,8 |
| Dow Corning® 1403⁶ | 3,0 |
| Euxyl® K 400 | 0,2 |
| Gluadin® W 20 | 2,0 |
| Wasser, entsalzt | ad 100 |

| | |
|---|---|
| ⁵ Emulsion eines aminofunktionellen Silikons (INCI-Bezeichnung: Amodimethicone (and) Trideceth-12 (and) Cetrimonium Chloride) (DOW CORNING) | |
| ⁶ Mischung aus Dimethylpolysiloxan und hydroxyterminiertem Dimethylpolysiloxan (INCI-Bezeichnung: Dimethicone (and) Dimethiconol) (DOW CORNING) | |

Die Formulierung wird nach der Haarwäsche auf das Haar aufgebracht und verbleibt auf dem Haar.

### 3. Haarpflegeformulierung

| | |
|---|---|
| Salcare®SC 96 | 5,0 |
| Celquat®H-100⁷ | 1,0 |
| Dow Corning® 939 | 0,8 |
| Dow Corning® 1403 | 3,5 |
| Euxyl® K 400 | 0,2 |
| Gluadin® W 20 | 2,0 |
| Wasser, entsalzt | ad 100 |

| | |
|---|---|
| ⁷ Copolymer aus Hydroxyethylcellulose und Diallyldimethylammoniumchlorid (93 % Aktivsubstanz; INCI-Bezeichnung: Polyquaternium-4) (NATIONAL STARCH) | |

Die Formulierung wird nach der Haarwäsche auf das Haar aufgebracht und verbleibt auf dem Haar.

### 4. Haarpflegeformulierung

| | |
|---|---|
| Salcare®SC 96 | 5,5 |
| Merquat®100⁸ | 0,8 |
| Dow Corning® 939 | 0,8 |
| Dow Corning® 1403 | 3,0 |
| Euxyl® K 400 | 0,2 |
| Gluadin® W 20 | 2,0 |
| Wasser, entsalzt | ad 100 |

| | |
|---|---|
| ⁸ Poly(dimethyldiallylammoniumchlorid) (40 % Aktivsubstanz; INCI-Bezeichnung: Polyquaternium-6) (CHEMVIRON) | |

Die Formulierung wird nach der Haarwäsche auf das Haar aufgebracht und verbleibt auf dem Haar.

### 5. Haarpflegeformulierung

| | |
|---|---|
| Salcare®SC 96 | 3,0 |
| Merquat®550⁹ | 0,8 |
| Dow Corning® 939 | 0,8 |
| Dow Corning® 1403 | 3,0 |
| Euxyl® K 400 | 0,2 |
| Gluadin® W 20 | 2,0 |
| Wasser, entsalzt | ad 100 |

| | |
|---|---|
| ⁹ Copolymer aus Acrylamid und Dimethyldiallylammoniumchlorid (8 % Aktivsubstanz in Wasser; INCI-Bezeichnung: Polyquaternium-7) (CHEMVIRON) | |

Die Formulierung wird nach der Haarwäsche auf das Haar aufgebracht und verbleibt auf dem Haar.

### 6. Haarpflegeformulierung

| | |
|---|---|
| Salcare®SC 96 | 5,5 |
| Celquat®H-100 | 1,0 |
| Gafquat®755¹⁰ | 1,0 |
| Dow Corning® 939 | 0,8 |
| Dow Corning® 1403 | 3,0 |
| Euxyl® K 400 | 0,2 |
| Gluadin® W 20 | 2,0 |
| Wasser, entsalzt | ad 100 |

| | |
|---|---|
| ¹⁰ Dimethylaminoethylmethacrylat-Vinylpyrrolidon-Copolymer, mit Diethylsulfat quaterniert (19 % Aktivsubstanz in Wasser; INCI-Bezeichnung: Polyquaternium-11) (GAF) | |

Die Formulierung wird nach der Haarwäsche auf das Haar aufgebracht und verbleibt auf dem Haar.

### 7. Haarpflegeformulierung

| | |
|---|---|
| Salcare®SC 96 | 5,0 |
| Polymer JR®400 | 0,5 |
| Luviquat®MS-370¹¹ | 3,0 |
| Dow Corning® 939 | 0,8 |
| Dow Corning® 1403 | 3,0 |
| Euxyl® K 400 | 0,2 |
| Gluadin® W 20 | 2,0 |
| Wasser, entsalzt | ad 100 |

| | |
|---|---|
| ¹¹Vinylpyrrolidon-Vinylimidazoliummethochlorid-Copolymer (ca. 40 % Aktivsubstanz; INCI-Bezeichnung: Polyquaternium-44) (BASF) | |

Die Formulierung wird nach der Haarwäsche auf das Haar aufgebracht und verbleibt auf dem Haar.

### 8. Haarpflegeformulierung

| | |
|---|---|
| Salcare®SC 96 | 5,5 |
| Merquat®100 | 1,0 |
| Luviquat®Hold¹² | 3,0 |
| Dow Corning® 939 | 0,8 |
| Dow Corning® 1403 | 3,0 |
| Euxyl® K 400 | 0,2 |
| Gluadin® W 20 | 2,0 |
| Wasser, entsalzt | ad 100 |

| | |
|---|---|
| ¹² quaterniertes Vinylcaprolactam-Vinylpyrrolidon-Vinylimidazol Copolymer (ca. 20 % Aktivsubstanz; INCI-Bezeichnung: Polyquatemium-46) (BASF) | |

Die Formulierung wird nach der Haarwäsche auf das Haar aufgebracht und verbleibt auf dem Haar.

### 9. Haar-Sprüh-Conditioner

| | |
|---|---|
| Salcare®SC 96 | 1,3 |
| Polymer JR®400 | 0,6 |
| Dow Corning® 939 | 0,8 |
| Dow Corning® 1403 | 0,4 |
| Euxyl® K 400 | 0,2 |
| Gluadin® W 20 | 2,0 |
| Wasser, entsalzt | ad 100 |

Die Formulierung wird nach der Haarwäsche auf das Haar aufgebracht und verbleibt auf dem Haar.

### 10. Pflegendes Haarreinigungsmittel

| | |
|---|---|
| Plantacare®1200 UP¹³ | 12,0 |
| Genagen®CAB¹⁴ | 8,0 |
| Cetiol®OE¹⁵ | 0,5 |
| Natriumbenzoat | 0,5 |
| Salcare®SC 96 | 1,5 |
| Polymer JR®400 | 1,0 |
| Dow Corning® 1403 | 3,0 |
| Wasser, entsalzt | ad 100 |

| | |
|---|---|
| ¹³ C₁₂₋₁₆-Alkyl-1,4-glucosid (ca. 50 % Aktivsubstanz in Wasser; INCI-Bezeichnung: Lauryl Glucoside) (HENKEL) | |
| ¹⁴ N,N-Dimethyl-N-kokosamidopropylammoniumacetobetain (ca. 30 % Aktivsubstanz in Wasser; INCI-Bezeichnung: Cocamidopropyl Betaine) (CLARIANT) | |
| ¹⁵ Dioctylether (INCI-Bezeichnung: Dicaprylyl Ether) (HENKEL) | |

### 11. Haarpflegemischung (rinse-off)

| | |
|---|---|
| Salcare®SC 96 | 2,0 |
| Polymer JR®400 | 0,5 |
| Cetearylalkohol | 4,5 |
| Dow Corning® 1403 | 4,5 |
| Cetyltrimethylammoniumchlorid | 0,5 |
| Euxyl® K 400 | 0,2 |
| Gluadin® W 20 | 2,0 |
| Wasser, entsalzt | ad 100 |

Die Formulierung wird nach der Haarwäsche auf das Haar aufgebracht und nach einer Einwirkzeit von 1 - 30 Minuten wieder ausgespült.

### 12. Haarpflegemischung (rinse-off)

| | |
|---|---|
| Salcare®SC 96 | 1,0 |
| Polymer JR®400 | 3,0 |
| Cetearylalkohol | 4,0 |
| Dow Corning® 1403 | 2,5 |
| Cetyltrimethylammoniumchlorid | 1,2 |
| Euxyl® K 400 | 0,2 |
| Gluadin® W 20 | 2,0 |
| Wasser, entsalzt | ad 100 |

Die Formulierung wird nach der Haarwäsche auf das Haar aufgebracht und nach einer Einwirkzeit von 1 - 30 Minuten wieder ausgespült.

### 12. Haarpflegemischung (rinse-off)

| | |
|---|---|
| Salcare®SC 96 | 1,5 |
| Polymer JR®400 | 0,2 |
| Merquat®100 | 0,8 |
| Cetearylalkohol | 5,0 |
| Dow Corning® 1403 | 3,0 |
| Cetyltrimethylammoniumchlorid | 1,6 |
| Euxyl® K 400 | 0,2 |
| Gluadin® W 20 | 2,0 |
| Wasser, entsalzt | ad 100 |

Die Formulierung wird nach der Haarwäsche auf das Haar aufgebracht und nach einer Einwirkzeit von 1 - 30 Minuten wieder ausgespült.

## Patentansprüche

1. Verfahren zur Behandlung keratinischer Fasern mit einem Haarfärbemittel oder einem direkt nach der Färbung angewendeten Nachbehandlungsmittel, das eine Wirkstoffkombination enthält, bestehend aus
- einem im Sinne der in der Beschreibung gegebenen Beigriffsdefinition "verdickend wirkenden" synthetischen Polymer (A), in Mengen von 0,1 - 10 Gew.-%, bezogen auf die Zubereitung, ausgewählt aus den Homopolymeren der allgemeinen Formel (I), 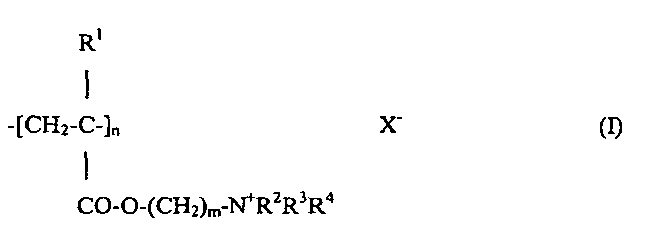 in der R¹ = -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymeren, bestehend im wesentlichen aus den in Formel (I) aufgeführten Monomereinheiten sowie nichtionogenen Monomereinheiten,
und
- mindestens einem kationischen Polymer (B), in Mengen von 0,01 - 5 Gew.-%, bezogen auf die Zubereitung, mit der Maßgabe, dass die Zubereitung zwingend ein Tensid enthält, wenn das Polymer (A) ein Copolymer ist, und mit der Maßgabe dass mindestens ein Polymer (B) keine verdickenden Eigenschaften aufweist oder dass mindestens ein Polymer (B) ein von dem Polymer (A) strukturell verschiedenes kationisches Polymer ist.

2. Zubereitung zur Behandlung keratinischer Fasern, insbesondere menschlicher Haare, **dadurch gekennzeichnet, dass** sie eine Wirkstoffkombination, bestehend aus
- einem im Sinne der in der Beschreibung gegebenen Begriffsdefinition "verdickend wirkenden" synthetischen Poymer (A), in Mengen von 0,1 - 10 Gew.-%, bezogen auf die Zubereitung, ausgewählt aus den Homopolymeren der allgemeinen Formel (I), 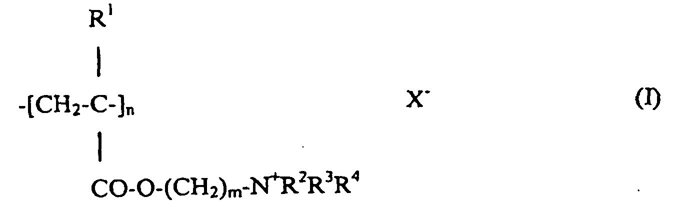 in der R¹ = -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymeren, bestehend im wesentlichen aus den in Formel (I) aufgeführten Monomereinheiten sowie nichtionogenen Monomereinheiten,
und
- mindestens einem kationischen Polymer (B), in Mengen von 0,01 - 5 Gew.-%, bezogen auf die Zubereitung, enthält mit der Maßgabe, dass die Zubereitung zwingend ein Tensid enthält, wenn das Polymer (A) ein Copolymer ist, und mit der Maßgabe, dass die Zubereitung weiterhin zwingend ein Proteinhydrolysat enthält, und mit der Maßgabe dass mindestens ein Polymer (B) keine verdickenden Eigenschaften aufweist oder dass mindestens ein Polymer (B) ein von dem Polymer (A) strukturell verschiedenes kationisches Polymer ist.

3. Zubereitung nach Anspruch 2, **dadurch gekennzeichnet, daß** in der Formel (I) R¹, R², R³ und R⁴ für eine Methylgruppe stehen und m den Wert 2 hat.

4. Zubereitung nach einem der Ansprüche 2 bis 3, **dadurch gekennzeichnet, daß** das Polymer (A) ein Homopolymer gemäß Formel (I) ist.

5. Zubereitung nach einem der Ansprüche 2 bis 4, **dadurch gekennzeichnet, daß** das Polymer (A) ein Copolymer ist, dessen nichtionogene Monomereinheiten ausgewählt sind aus der Gruppe, die von Acrylamid, Methacrylamid, Acrylsäure-C₁₋₄-Alkylestern und Methacrylsäure-C₁₋₄-Alkylestern gebildet wird.

6. Zubereitung nach Anspruch 5, **dadurch gekennzeichnet, daß** die nichtionogene Monomereinheit Acrylamid ist.

7. Zubereitung nach einem der Ansprüche 2 bis 6, **dadurch gekennzeichnet, daß** das kationische Polymer (B) ein kationisches Cellulose-Derivat ist.

8. Zubereitung nach einem der Ansprüche 2 bis 7, **dadurch gekennzeichnet, daß** sie weiterhin mindestens ein Silikon enthält.

9. Zubereitung nach Anspruch 8, **dadurch gekennzeichnet, daß** sie eine Mischung aus einem flüchtigen und einem nichtflüchtigen Silikon enthält.

10. Zubereitung nach einem der Ansprüche 2 bis 9, **dadurch gekennzeichnet, daß** sie ein Proteinhydrolysat enthält.

11. Zubereitung nach einem der Ansprüche 2 bis 10, **dadurch gekennzeichnet, daß** sie weiterhin ein Tensid enthält.

## Claims

1. Method of treating keratin fibres with a hair colorant or an aftertreatment agent applied directly after colouring which comprises an active ingredient combination consisting of
- a synthetic polymer (A) which has a "thickening effect" in the sense of the definition of terms given in the description, in amounts of 0.1 - 10% by weight, based on the preparation, chosen from the homopolymers of the general formula (I), 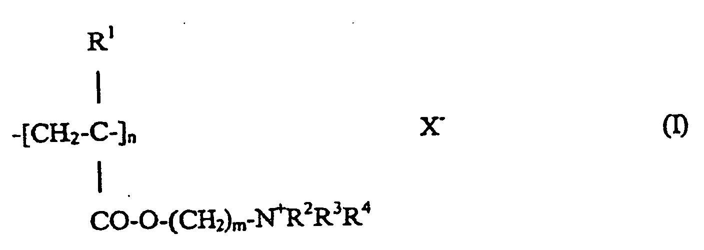 in which R¹ = -H or -CH₃, R², R³ and R⁴, independently of one another, are chosen from C₁₋₄-alkyl, -alkenyl or -hydroxyalkyl groups, m = 1, 2, 3 or 4, n is a natural number and X⁻ is a physiologically compatible organic or inorganic anion, and copolymers consisting essentially of the monomer units detailed in formula (I), and nonionogenic monomer units,
and
- at least one cationic polymer (B), in amounts of 0.01 - 5% by weight, based on the preparation, with the proviso that the preparation necessarily comprises a surfactant if the polymer (A) is a copolymer, and with the proviso that at least one polymer (B) has no thickening properties or that at least one polymer (B) is a cationic polymer which is structurally different from the polymer (A).

2. Preparation for treating keratin fibres, in particular human hair, **characterized in that** it comprises an active ingredient combination consisting of
- a synthetic polymer (A) which has a "thickening effect" in the sense of the definition of terms given in the description, in amounts of 0.1 - 10% by weight, based on the preparation, chosen from the homopolymers of the general formula (I), 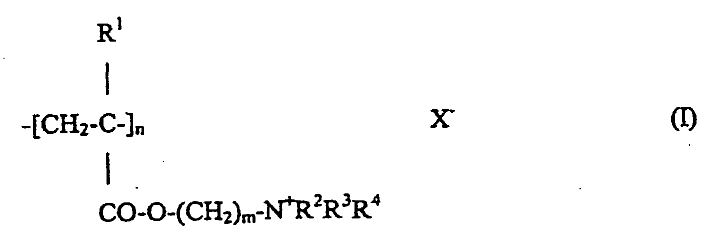 in which R¹ = -H or -CH₃, R², R³ and R⁴, independently of one another, are chosen from C₁₋₄-alkyl, -alkenyl or -hydroxyalkyl groups, m = 1, 2, 3 or 4, n is a natural number and X⁻ is a physiologically compatible organic or inorganic anion, and copolymers consisting essentially of the monomer units detailed in formula (I), and nonionogenic monomer units,
and
- at least one cationic polymer (B), in amounts of 0.01 - 5% by weight, based on the preparation, with the proviso that the preparation necessarily comprises a surfactant if the polymer (A) is a copolymer, and with the proviso that the preparation also necessarily comprises a protein hydrolysate, and with the proviso that at least one polymer (B) has no thickening properties or that at least one polymer (B) is a cationic polymer which is structurally different from the polymer (A).

3. Preparation according to Claim 2, **characterized in that**, in the formula (I), R¹, R², R³ and R⁴ are a methyl group and m has the value 2.

4. Preparation according to one of Claims 2 to 3, **characterized in that** the polymer (A) is a homopolymer according to formula (I).

5. Preparation according to one of Claims 2 to 4, **characterized in that** the polymer (A) is a copolymer whose nonionogenic monomer units are chosen from the group which is formed by acrylamide, methacrylamide, acrylic C₁₋₄-alkyl esters and methacrylic C₁₋₄-alkyl esters.

6. Preparation according to Claim 5, **characterized in that** the nonionogenic monomer unit is acrylamide.

7. Preparation according to one of Claims 2 to 6, **characterized in that** the cationic polymer (B) is a cationic cellulose derivative.

8. Preparation according to one of Claims 2 to 7, **characterized in that** it also comprises at least one silicone.

9. Preparation according to Claim 8, **characterized in that** it comprises a mixture of a volatile silicone and a nonvolatile silicone.

10. Preparation according to one of Claims 2 to 9, **characterized in that** it comprises a protein hydrolysate.

11. Preparation according to one of Claims 2 to 10, **characterized in that** it also comprises a surfactant.

## Revendications

1. Procédé de traitement de fibres kératiniques avec un colorant pour cheveux ou un produit de post-traitement utilisé directement après coloration, qui contient une combinaison de substances actives, constituée
- d'un polymère de synthèse « à action épaississante » (A), au sens de la définition de ce concept donnée dans la description, à des quantités de 0,1 à 10 % en poids, par rapport à la préparation, choisi parmi les homopolymères de formule générale (I), 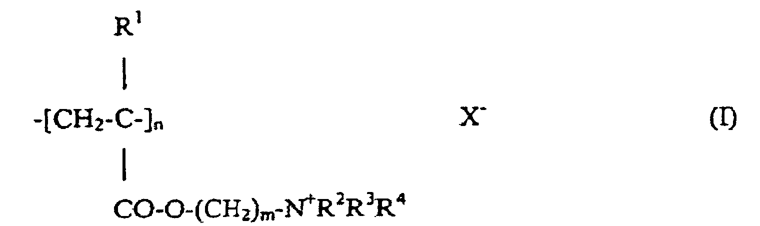 dans laquelle R¹ = -H ou -CH₃, R², R³ et R⁴ sont choisis indépendamment l'un de l'autre parmi les groupes alkyle, alcényle ou hydroxyalkyle en C₁ à C₄, m = 1, 2, 3 ou 4, n représente un nombre naturel et X⁻ est un anion organique ou inorganique physiologiquement acceptable, ainsi que les copolymères constitués pour l'essentiel d'unités monomères mentionnées dans la formule (I) ainsi que d'unités monomères non ionogènes,
et
- d'au moins d'un polymère cationique (B), à des quantités de 0,01 à 5 % en poids, par rapport à la préparation, sous réserve que la préparation contient obligatoirement un agent tensioactif lorsque le polymère (A) est un copolymère, et sous réserve qu'au moins un polymère (B) ne présente pas de propriétés épaississantes, ou qu'au moins un polymère (B) est un polymère cationique structurellement différent du polymère (A).

2. Préparation pour le traitement de fibres kératiniques, en particulier de cheveux humains, **caractérisée en ce qu'**elle contient une combinaison de substances actives constituée
- d'un polymère de synthèse « à action épaississante » (A), au sens de la définition de ce concept donnée dans la description, à des quantités de 0,1 à 10 % en poids, par rapport à la préparation, choisi parmi les homopolymères de formule générale (I), 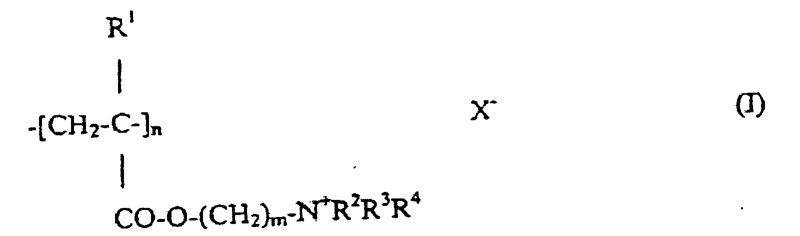 et
- d'au moins un polymère cationique (B), à des quantités de 0,01 à 5 % en poids, par rapport à la préparation, sous réserve que la préparation contienne obligatoirement un agent tensioactif lorsque le polymère (A) est un copolymère, et sous réserve que la préparation contienne en outre obligatoirement un hydrolysat de protéine, ainsi que sous réserve qu'au moins un polymère (B) ne présente pas de propriétés épaississantes, ou qu'au moins un polymère (B) est un polymère cationique structurellement différent du polymère (A).

3. Préparation selon la revendication 2, **caractérisée en ce que** dans la formule (I) R¹, R², R³ et R⁴ représentent un groupe méthyle et m a la valeur 2.

4. Préparation selon l'une des revendications 2 à 3, **caractérisée en ce que** le polymère (A) est un homopolymère selon la formule (I).

5. Préparation selon l'une des revendications 2 à 4, **caractérisée en ce que** le polymère (A) est un copolymère dont les unités monomères non ionogènes sont choisies dans le groupe formé par l'acrylamide, le méthacrylamide, les esters acryliques de l'acide acrylique en C₁ à C₄ et les esters alkyliques de l'acide méthacrylique en C₁ à C₄.

6. Préparation selon la revendication 5, **caractérisée en ce que** l'unité monomère non ionogène est l'acrylamide.

7. Préparation selon l'une des revendications 2 à 6, **caractérisée en ce que** le polymère cationique (B) est un dérivé de cellulose cationique.

8. Préparation selon l'une des revendications 2 à 7, **caractérisée en ce qu'**elle contient en outre au moins une silicone.

9. Préparation selon la revendication 8, **caractérisée en ce qu'**elle contient un mélange d'une silicone volatile et d'une silicone non volatile.

10. Préparation selon l'une des revendications 2 à 9, **caractérisée en ce qu'**elle contient un hydrolysat de protéine.

11. Préparation selon l'une des revendications 2 à 10, **caractérisée en ce qu'**elle contient en outre un agent tensioactif.
